# EUROPÄISCHE PATENTSCHRIFT

(11) **EP 2 465 569 B1**
(45) Veröffentlichungstag und Bekanntmachung des Hinweises auf die Patenterteilung: **25.05.2016**
(21) Anmeldenummer: 11191736.5
(22) Anmeldetag: 02.12.2011
(51) Int. Cl.: A61N 1/05

(54) **Implantierbares Gerät**
Implantable device
Appareil implantable

(30) Priorität: 17.12.2010 US 424079 P
(43) Veröffentlichungstag der Anmeldung: 20.06.2012
(73) Patentinhaber: BIOTRONIK SE & Co. KG, 12359 Berlin (DE)
(72) Erfinder: Weiss, Ingo, 12435 Berlin (DE); Knorr, Stefan, 10119 Berlin (DE); Maxfield, Michelle, 10967 Berlin (DE); Friedrich, Michael, 14532 Kleinmachnow (DE)
(74) Vertreter: Galander, Marcus

(56) Entgegenhaltungen:
- WO-A1-2008/115426
- WO-A2-2007/047966
- US-A1- 2008 132 985
- US-A1- 2009 099 555

## Beschreibung

Die Erfindung betrifft ein permanent oder temporär implantierbares Gerät mit einem langgestreckten elektrischen Leiter.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Deshalb können Herzschrittmacherpatienten heutzutage in der Regel nicht oder nur eingeschränkt in einem Kernspintomografen untersucht werden.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Elektrodenpole sind typischerweise in Form eines Rings um die Elektrodenleitung mit einer elektrisch leitenden Oberfläche oder in Form einer Spitzen- oder Tippelektrode am distalen Ende der Elektrodenleitung vorgesehen. Die Elektrodenpole sind über eine oder mehrere elektrische Leitungen mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leitungen, die einen oder mehrere der Elektrodenpole mit einem oder mehreren der Kontakte elektrisch verbinden. Diese elektrischen Leitungen können einerseits zur Übertragung von Stimulationsimpulsen zu den Elektrodenpolen oder zur Übertragung mittels der Elektrodenpole aufgenommener elektrischer Signale zum proximalen Ende der Elektrodenleitung genutzt werden und werden im Verlauf der weiteren Beschreibung als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen kann.

Dokument US-A-2008/132985 offenbart den nächsliegenden Stand der Technik.

Der Erfindung liegt die Aufgabe zugrunde, ein Gerät zu schaffen, welches das zuvor beschriebene Problem löst.

Erfindungsgemäß wird diese Aufgabe durch ein temporär oder permanent implantierbares medizinisches Gerät gemäß Anspruch 1 gelöst.

Bei dem erfindungsgemäßen medizinischen Gerät erfährt die mindestens eine erste Ader des ersten Funktionsleiters einen anderen elektromagnetischen Einfluss äußerer Felder als die mindestens eine zweite Ader desselben Funktionsleiters. Denn nur die mindestens eine erste Ader ist durch die erste Kopplung mit einem zweiten Funktionsleiter demjenigen elektromagnetischen Einfluss äußerer Felder ausgesetzt, den auch der zweite Funktionsleiter erfährt. Die mindestens eine zweite Ader des ersten Funktionsleiters erfährt zwar zu jedem Zeitpunkt ebenso einen elektromagnetischen Einfluss des betreffenden äußeren Feldes, jedoch unterscheidet sich dieser Einfluss aufgrund der fehlenden ersten Kopplung der zweiten Ader(n) mit dem zweiten Funktionsleiter und der inhärenten räumlichen Trennung der zweiten Ader(n) vom zweitem Funktionsleiter innerhalb der elektrischen Leitung. Solche Unterschiede machen sich bei induzierten hochfrequenten elektromagnetischen Strömen typischerweise durch unterschiedliche Phasenlagen bemerkbar.

Dies macht sich das medizinische Gerät der vorliegenden Erfindung zunutze, indem durch die zweite Kopplung eine zumindest in Teilen destruktive Überlagerung der in der mindestens einen ersten Ader und der in der mindestens einen zweiten Ader geführten elektromagnetischen Wellen geschaffen wird. Diese destruktive Überlagerung sorgt unter dem Einfluss eines hochfrequenten magnetischen Wechselfeldes, wie es in Magnetresonanz-Geräten zum Einsatz kommt, für eine Reduzierung der im ersten Funktionsleiter induzierten hochfrequenten Ströme am Überlagerungsort. Auf diese Weise sorgen die erste und die zweite Kopplung insgesamt für einen reduzierte Stromstärke induzierter hochfrequenter Ströme auf dem ersten Funktionsleiter. Dies reduziert oder eliminiert im implantierten Zustand des medizinischen Geräts die unerwünschte Erwärmung des Körpergewebes, welches die mit dem ersten Funktionsleiter verbundene Funktions-Elektrode umgibt.

Nachfolgend werden Ausführungsbeispiele des medizinischen Geräts der vorliegenden Erfindung erläutert. Die zusätzlichen Merkmale der einzelnen Ausführungsbeispiele können miteinander zur Bildung weiterer Ausführungsformen des medizinischen Geräts kombiniert werden, soweit sie nicht ausdrücklich als einander ausschließende Alternativen beschrieben sind.

Die langgestreckte elektrische Leitung ist in bevorzugten Ausführungsbeispielen eine temporär oder permanent implantierbare Elektrodenleitung zur Verbindung eines oder mehrerer Funktions-Elektrodenpole mit einem Steuergerät, wie beispielsweise dem Steuergerät eines implantierbaren Herzschrittmachers oder eines implantierbaren Defibrillators. Die langgestreckte Leitung bildet jedoch als solche schon ein medizinisches Gerät im Sinne der vorliegenden Beschreibung.

Die Auslegung der Geometrien und Dimensionierung der Leiterabstände (insbesondere die Festlegung der jeweiligen "Nähe") erfolgt vorzugsweise so, dass eine Minimierung der Erwärmung der Elektrodenpole beim Betrieb dieses Implantats in einem von magnetischen Wechselfeldern belasteten Umfeld erzielt wird.

Die erste und die zweite Kopplung sind vorzugsweise so konfiguriert, dass sie am mit dem ersten Funktionsleiter verbundenen Funktionselektrodenpol eine zumindest teilweise destruktive Interferenz von elektromagnetischen Radiofrequenz-Wellen bewirken, welche in der mindestens einen ersten Ader und der mindestens einen zweiten Ader des ersten Funktionsleiters geführt sind. Mit anderen Worten, der oben genannte zweite Längsabschnitt, in dem die zweite Kopplung vorliegt, kann am Ort des Funktionselektrodenpols liegen. Am Funktions-Elektrodenpol ist eine Reduzierung induzierter hochfrequenter Ströme im Sinne der Erfindung besonders wirksam, weil dort im implantierten Zustand des elektrischen Leiters eine besonders starke thermische Kopplung mit dem Körpergewebe gegeben ist.

Es hat sich als vorteilhaft herausgestellt, wenn , bei die mindestens eine erste Ader und die mindestens eine zweite Ader des ersten Funktionsleiters in mindestens einem dritten, vom ersten und zweiten verschiedenen und mit einem geringeren Abstand als diese von einem proximalen Ende der elektrischen Leitung angeordneten Längsabschnitt eine dritte Kopplung aufweisen, die die geeignet ist, in der mindestens einen zweiten Ader geführte elektromagnetische Radiofrequenz-Wellen zumindest teilweise in die mindestens eine erste Ader des ersten Funktionsleiters einzukoppeln. Die zusätzliche Ankopplung im dritten Längsabschnitt, der insbesondere ein proximaler Ankopplungspunkt sein kann, schafft dort einen elektrischen Referenzpunkt. Mit andern Worten wird durch das Vorsehen dieses zusätzlichen Ankopplungspunktes eine Reflexion der auf der mindestens einen ersten Ader des Funktionsleiters laufenden elektromagnetischen Wellen so eingestellt dass diese am zweiten Längsabschnitt des Funktionsleiters eine solche Amplituden- und Phasenlage aufweisen, die den gewünschten Auslöschungseffekt besonders gut bewirken kann.

Alternativ kann zu diesem Zweck eine Kreuzkopplung vorgesehen sein. Das heißt, bei die mindestens eine erste Ader und mindestens eine Ader des zweiten Funktionsleiters weisen in mindestens einem dritten, vom ersten und zweiten verschiedenen und mit einem geringeren Abstand als diese von einem proximalen Ende der elektrischen Leitung angeordneten Längsabschnitt eine dritte Kopplung auf, die die geeignet ist, in der mindestens einen Ader des zweiten Funktionsleiters geführte elektromagnetische Radiofrequenz-Wellen zumindest teilweise in die mindestens eine erste Ader des ersten Funktionsleiters einzukoppeln.

In verschiedenen Ausführungsformen des medizinischen Geräts können unterschiedliche Formen einer Kopplung mit einem Phasenschieber-Bauelement erzielt werden. Dazu ist die mindestens eine erste Ader des ersten Funktionsleiters oder die mindestens eine zweite Ader in der elektrischen Leitung mindestens einem Phasenschieber-Bauelement zugeführt, das dem Elektrodenpol vorgeschaltet ist. Das Phasenschieber-Bauelement dient dazu, mit vorzugsweise passiven Bauelementen die Phasenlage der in die erste Ader eingekoppelten elektromagnetischen Welle so einzustellen, dass eine möglichst destruktive Interferenz am Ort des Elektrodenpols erzielt werden kann.

Der Anschluss der mindestens einen ersten Ader der ersten Funktionsleitung an die Elektrodenpole und/oder einen proximal vorgesehenen Stecker zum Anschluss an ein Steuergerät erfolgt in einer Variante dieser Ausführungsform nicht galvanisch, sondern wird über jeweils eine erste Phasenschieber-Schaltung realisiert. Diese Schaltung ist vorzugsweise ausschließlich mit passiven, also insbesondere resistiven, induktiven und oder kapazitiven Bauelementen ausgeführt, oder nutzt geeignet geformte Materialien, die die Wirkung dieser passiven Bauteile erzeugen.

Alternativ oder zusätzlich ist der entsprechende Anschluss der mindestens einen zweiten Ader der ersten Funktionsleitung an den Funktionselektrodenpol und/ an einen Stecker mit einer entsprechenden zweiten Phasenschieber-Schaltung verbunden, für deren Ausführung dasselbe gilt wie es im letzten Absatz für die erste Phasenschieberschaltung beschrieben wurde. Wenn beide Adern einer jeweiligen Phasenschieber-Schaltung zugeführt werden, kann das Überlagern der Signale auf der ersten und zweiten Ader gewichtet gemischt werden, um die Auslöschung möglichst genau einstellen zu können.

Eine solche Phasenschieber-Schaltung kann in einer Weiterbildung dieser Variante konfiguriert sein, zusätzlich eine abschwächende Wirkung auf die elektromagnetischen Wellen auszuüben.

Ergänzend oder alternativ zu passiven Bauelementen können in einer solchen Phasenschieber-Schaltung auch aktive Bauelemente wie Schalter, beispielsweise als Halbleiterbauelemente realisiert, verwendet werden.

Eine besonders einfache Realisierung der Phasenschieberschaltung bildet ein Zweipol einer Impedanz Z, die mit der betreffenden Ader in Reihe geschaltet ist. Beispielsweise ist die Kopplung der mindestens einen ersten und der mindestens einen zweiten Ader kapazitiv. Dies ist durch eine Isolierung einer der Adern realisierbar. Vorzugsweise ist die Kapazität größer als 1 Pikofarad.

Die Herstellung der Kopplungen ist herstellungstechnisch günstig, wenn die ersten Adern der ersten Funktionsleitung, egal wie diese realisiert ist, ob beispielsweise als Wendel oder als Seil, in gleicher Weise wie die der zweiten Zuleitung ausgebildet ist, damit sie auf einfache Weise zur Herstellung der jeweiligen Kopplung in den ersten, zweiten und gegebenenfalls dritten Längsabschnitten nahe bei einander geführt sein können (z.B. parallel gewendelt).

Die Kopplung der mindestens einen ersten und der mindestens einen zweiten Ader erfolgt in einem anderen Ausführungsbeispiel durch einen elektrischen Schleifkontakt. Ein Schleifkontakt als mechanische Ausführung der Kontaktierung kann mobil, also drehbar, verschiebbar und/oder drehbar mit Vorschub gestaltet werden, was die mechanische Flexibilität des elektrischen Leiters als Ganzes fördert.

Die mindestens eine erste Ader des ersten Funktionsleiters kann mit mindestens einer Ader des zweiten Funktionsleiters zur Herstellung der ersten Kopplung verdrillt sein.

Die Adern sind in einer anderen Ausführungsform jeweils als Wendeln ausgeführt. In einer Variante dieser Ausführungsform ist die mindestens eine erste Ader des ersten Funktionsleiters zur Herstellung der ersten Kopplung wendelförmig um mindestens eine Ader des zweiten Funktionsleiters gewickelt ist und hüllt sie ein.

Es ist auch möglich, eine der Adern als Wendel und die andere Ader zumindest abschnittsweise als Seil auszuführen und die seilförmige Wendel zur Herstellung der Kopplung im Hohlraum der Wendel zu führen.

Das vorstehend für die erste bzw. zweite Kopplung Gesagte lässt sich ohne weiteres auf jeweils die anderen erwähnten Kopplungen, also die zweite bzw. die erste und die dritte Kopplung übertragen.

Die mindestens eine erste Ader des ersten Funktionsleiters und mindestens eine Ader des zweiten Funktionsleiters sind in einer Ausführungsform aus unterschiedlichen Materialien gefertigt. Als Materialien eigenen sich beispielsweise kobaltfreie Materialien, etwa Zirkonium, Tantal, DFT oder MP35N.

Weitere Ausführungsbeispiele des medizinischen Geräts werden nachfolgend anhand der Figuren erläutert. Es zeigen:
- Fig. 1: Ausführungsbeispiele medizinischer Geräte in Form eines Herzschrittmachers und einer daran angeschlossenen Elektrodenleitung;
- Fig. 2: eine schematische Darstellung einer Elektrodenleitung nach dem Stand der Technik
- Fig. 3: eine schematische Darstellung eines Ausführungsbeispiels einer erfindungsgemäßen Elektrodenleitung;
- Fig. 4: eine schematische Darstellung einer ersten Variante des Ausführungsbeispiels der Fig. 3; und
- Fig. 5: eine schematische Darstellung eines weiteren Ausführungsbeispiels einer erfindungsgemäßen Elektrodenleitung;

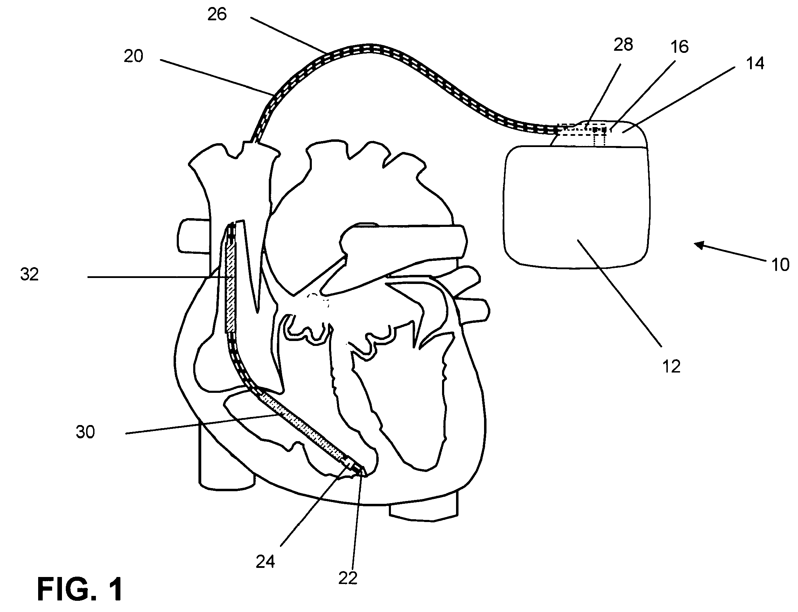

Fig. 1 zeigt als Beispiele implantierbarer medizinischer Geräte einen implantierbaren Herzstimulator 10 und eine daran angeschlossene implantierbare Elektrodenleitung 20.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tip-Elektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tip-Elektrode 22 und die Ringelektrode 24, im Anwendungsfall die Elektrodenleitung 20, im Apex eines rechten Ventrikels eines Herzens befinden.

Sowohl die Tip-Elektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren.

Wie weiter unten näher erläutert wird, können die elektrischen Leiter 26 in der Elektrodenleitung 20 in unterschiedlichen Längsabschnitten als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden, werden im Rahmen dieses Textes auch als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Funktionsleiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist. Weiterhin können solche Elektrodenleitungen unter fachüblicher Anpassung an die speziellen Erfordernisse des jeweiligen Anwendungsgebiets auch zur Stimulation und Ableitung von Signalen an Nerven, Gehirn, und anderen Organen oder für die Zuleitung von implantierbaren Sensoren Verwendung finden.

Nachfolgend werden einige Ausführungsbeispiele für die erfindungsgemäße Gestaltung der Elektrodenleitung 20 erläutert. Zuvor wird auf eine Gestaltung gemäß dem Stand der Technik eingegangen.

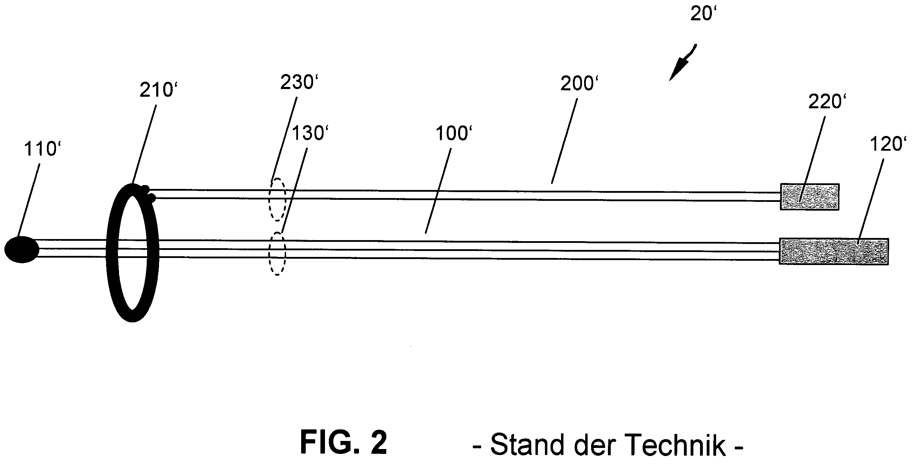

Fig. 2 zeigt eine schematische Darstellung einer solchen Elektrodenleitung 20' nach dem Stand der Technik. Die Elektrodenleitung hat an ihrem distalen Ende zwei Funktions-Elektrodenpole in Form eines Tip-Elektrodenpols 110' und eines Ring-Elektrodenpols 210'. Für die Zuleitung oder Ableitung von Signalen zum bzw. vom Tip-Elektrodenpol 110' ist eine Funktionsleitung 100' mit dem Tip-Elektrodenpol 110' verbunden, die im vorliegenden Beispiel 3 Adern aufweist. Proximal ist sie mit einem Steckerpol 120' verbunden, der an ein Steuergerät eines Herzschrittmachers anschließbar ist.

Für die Zuleitung oder Ableitung von Signalen zum bzw. vom Ring-Elektrodenpol 210' ist eine Funktionsleitung 200' mit dem Ring-Elektrodenpol 210' verbunden, die im vorliegenden Beispiel 2 Adern aufweist. Proximal ist die Funktionsleitung 200' mit einem Steckerpol 220' verbunden, der ebenfalls an das Steuergerät des Herzschrittmachers anschließbar ist. Die Adern der Funktionsleitung 100' sind nahe bei einander geführt, was durch einen gestrichelten Kreis 130' angedeutet ist. Ebenso sind die Adern der Funktionsleitung 200' nahe bei einander geführt, was durch einen gestrichelten Kreis 230' angedeutet ist.

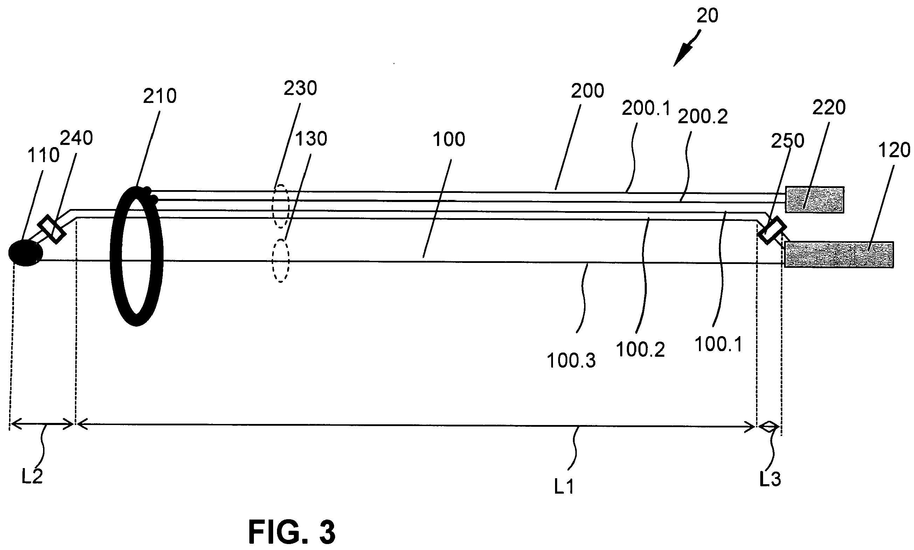

Fig. 3 zeigt eine entsprechend schematische Darstellung eines Ausführungsbeispiels einer erfindungsgemäßen Elektrodenleitung.

Der Einfachheit der Darstellung halber werden hier wie in den nachfolgenden Figuren weitgehend identische Bezugszeichen für einander entsprechende Strukturelemente verwendet, abgesehen vom oben verwendeten Hochstrich am Ende der Bezugszeichen, der in den Figuren 3 bis 5 zur Kennzeichnung des Unterschieds zwischen dem Stand der Technik und Ausführungsbeispielen der Erfindung fortgelassen wird.

Die hier beschriebenen Ausführungsbeispiele lehnen sich der Einfachheit der Darstellung halber in einigen Merkmalen an die bekannte Ausführung an. Dies erfolgt jedoch nur zur Verdeutlichung des Unterschieds der erfindungsgemäßen Ausführungsformen und soll nicht bedeuten, dass solche Merkmale notwendig für die Realisierung der Erfindung sind. Beispielsweise übernehmen die Fig. 3 bis 5 die Anzahl der Adern der Funktionsleiter 100 und 200 vom Beispiel der Fig. 2. Die tatsächliche Anzahl der Adern kann jedoch auch über der gezeigten Anzahl liegen. Auch kann der Funktionsleiter 100 beispielsweise nur 2 A-dem haben.

Die nachfolgende Beschreibung konzentriert sich auf Unterschiede der Elektrodenleitung der vorliegenden Ausführungsbeispiele gegen über der bekannten Ausführungsform der Fig. 2.

Von den drei Adern 100.1 bis 100.3 des ersten Funktionsleiters 100 sind im vorliegenden Ausführungsbeispiel die Adern 100.1 und 100.2 als erste Adern im Sinne der einleitenden Erfindungsbeschreibung von der Ader 100.3, die im Sinne der einleitenden Erfindungsbeschreibung eine zweite Ader bildet, ein einem ersten Längsabschnitt L1 entfernt geführt. In diesem ersten Längsabschnitt sind die nahe an Adern 200.1 und 200.2 der zweiten Funktionsleitung 200 geführt, wodurch eine induktive und kapazitive Einkopplung von elektromagnetischen Radiofrequenz-Wellen von der zweiten Funktionsleitung 200 in die beiden Adern 100.1 und 100.2 realisiert ist, als erste Kopplung 230 im Sinne der obigen Beschreibung.

In einem nahe dem distalen Ende des ersten Funktionsleiters 100 angeordneten Phasenschieber 240 werden die Phasen eingekoppelten Wellen verschoben. Dies erfolgt zur Erzielung einer destruktiven Interferenz bei Einkopplung der auf den Adern 100.1 und 100.2 geführten Wellen in die Ader 100.3 durch eine zweite Kopplung in einem zweiten Längsabschnitt L2 an der Tip-Elektrode 110.

Zur Bildung einer Phasenreferenz ist in einem dritten, proximalen Längsabschnitt L3 ein zusätzlicher Phasenschieber 250 vorgesehen.

Bei dem vorliegenden medizinischen Gerät erfahren die Adern 100.1 und 100.2 des ersten Funktionsleiters 100 einen anderen elektromagnetischen Einfluss äußerer Felder als die mindestens Ader 100.3 desselben Funktionsleiters. Denn nur die Adern 100.1 und 100.2 sind durch die erste Kopplung 230 mit dem zweiten Funktionsleiter 200 demjenigen elektromagnetischen Einfluss äußerer Felder ausgesetzt, den auch der zweite Funktionsleiter 200 erfährt. Die Ader 100.3 des ersten Funktionsleiters 100 erfährt zwar zu jedem Zeitpunkt ebenso einen elektromagnetischen Einfluss des betreffenden äußeren Wechselfeldes, jedoch unterscheidet sich dieser Einfluss aufgrund der fehlenden ersten Kopplung 230 der Ader 100.3 mit dem zweiten Funktionsleiter 200 und der inhärenten räumlichen Trennung der Ader 100.3 vom zweitem Funktionsleiter 200 innerhalb der elektrischen Leitung 20. Solche Unterschiede machen sich bei induzierten hochfrequenten elektromagnetischen Strömen durch unterschiedliche Phasenlagen bemerkbar, die für die destruktive Interferenz am Tip-Elektrodenpol 110 genutzt werden.

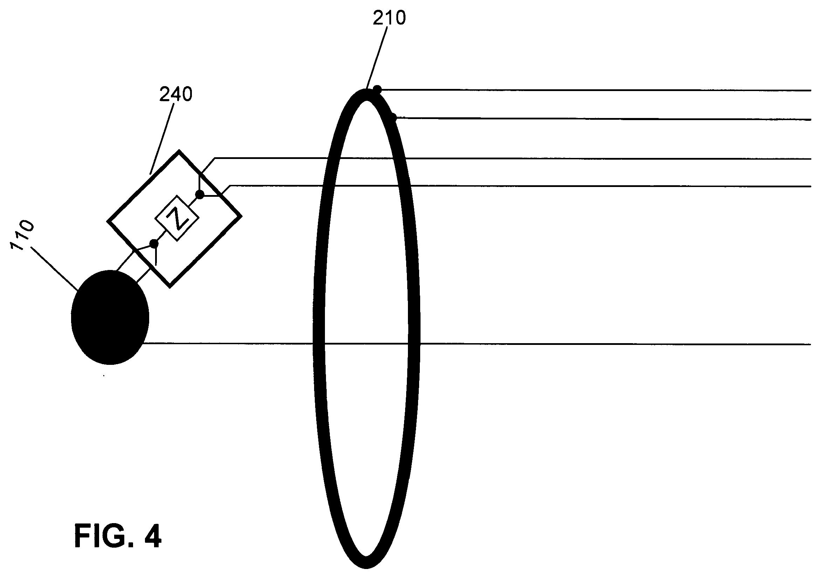

Fig. 4 zeigt eine schematische Darstellung einer ersten Variante des Ausführungsbeispiels der Fig. 3, bei der der Phasenschieber 240 eine besondere Ausprägung als Zweipol mit einer Impedanz Z, die mit der betreffenden den Adern 100.1 und 100.2 in Reihe geschaltet ist. Die Impedanz Z kann beispielsweise eine Kapazität größer als 1 Pikofarad sein. Alternative Realisierungen von des Phasenschieber 240/250 haben eine Impedanz Z in Form eines ohmschen Widerstands R mit R<200Ohm oder mit R>1000Ohm, bzw. an einem Ende der Adern R<200Ohm und am anderen End der Adern R>1000Ohm.

Der Phasenschieber 240 bzw. 250 kann die Impedanz Z alternativ in Form einer LC-Schaltung oder in Form einer RLC enthalten. Hier wird |Z|<200ohm oder |Z|>1000ohm frequenzabhängig realisiert, beispielsweise als Tiefpass, Hochpass, Bandpass, oder Bandsperre.

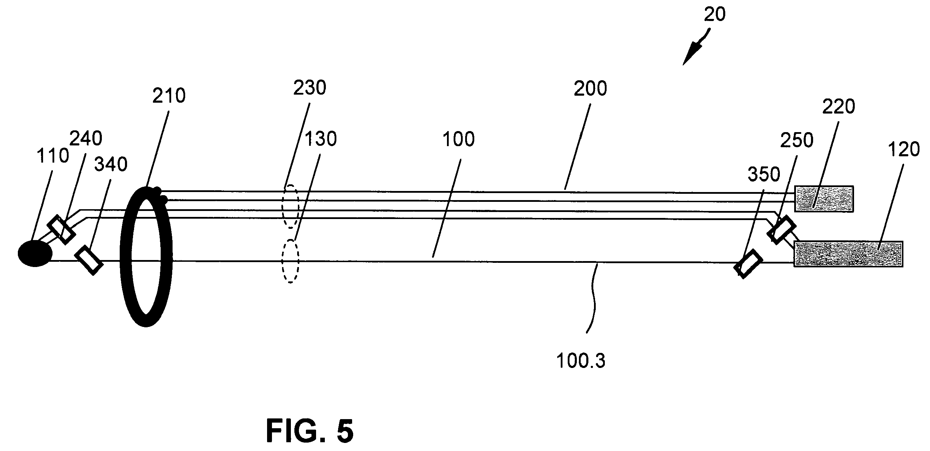

Fig. 5eine schematische Darstellung eines weiteren Ausführungsbeispiels einer erfindungsgemäßen Elektrodenleitung. Diese Variante unterscheidet sich von der der Fig. 3 allein dadurch, dass auch die Ader 100.3 mit Phasenschiebern 340 und 350 versehen ist.

Zur Verbesserung des Effektes kann das Lumen der Ummantelung der Elektrodenleitung 20 mit Isolatormaterial gefüllt sein. Die Funktionsleiter können im Innern des Isolatormaterials geführt werden. Die Adern können dann auch ohne eine individuelle Isolierung im Elektrodenleiter geführt werden. Ein zusätzlicher innerer Isolationsmantel kann zur Platzersparnis fortgelassen werden.

Bei wendelförmiger Führung der Adern der Funktionsleiter kann zwischen inneren und äußeren Wendeln eine Silikonfüllung verwendet werden. Zusätzlich kann eine Silikonummantelung verwendet werden, was eine gute Energieeinkopplung in umgebendes Gewebe bewirkt.

## Patentansprüche

1. Temporär oder permanent implantierbares medizinisches Gerät mit mindestens einer langgestreckten elektrischen Leitung mit mindestens zwei Funktionsleitern, die jeweils mehradrig sind und von denen jeder Funktionsleiter mit mindestens einem jeweiligen Funktionselektrodenpol zur Abgabe von Therapiesignalen oder zur
Erfassung von Diagnosesignalen verbunden ist, bei dem
- ein erster der Funktionsleiter (100) mindestens eine erste Ader aufweist, die im Verlauf der Längserstreckung des ersten Funktionsleiters in mindestens einem ersten Längsabschnitt, der nur ein Teilstück der Längserstreckung des Funktionsleiters bildet, eine erste Kopplung mit einem zweiten der Funktionsleiter (200) aufweist, die konfiguriert ist, im zweiten Funktionsleiter geführte elektromagnetische Radiofrequenz-Wellen zumindest teilweise in die mindestens eine erste Ader des ersten Funktionsleiters und, ***aufgrund einer inhärenten räumlichen Trennung mindestens einer von der ersten verschiedenen zweiten Ader des ersten Funktionsleiters vom zweiten Funktionsleiter,*** nicht in ***die*** mindestens eine von der ersten verschiedenen zweiten Ader des ersten Funktionsleiters einzukoppeln, und bei dem
- die mindestens eine erste Ader im Verlauf der Längserstreckung des ersten Funktionsleiters in mindestens einem vom ersten Längsabschnitt verschiedenen zweiten Längsabschnitt eine zweite Kopplung mit der mindestens einen zweiten Ader des ersten Funktionsleiters aufweist, die geeignet ist, in der mindestens einen ersten Ader geführte elektromagnetische Radiofrequenz-Wellen zumindest teilweise in die mindestens eine zweite Ader des ersten Funktionsleiters einzukoppeln, ***und wobei***
- ***die mindestens eine erste Ader des ersten Funktionsleiters im ersten Längsabschnitt mit einem geringeren, zur Herstellung der ersten Kopplung geeigneten Abstand vom zweiten Funktionsleiter geführt ist als ausserhalb des ersten Längsabschnitts des ersten Funktionsleiters.***

2. Medizinisches Gerät nach ***Anspruch 1***, bei dem die erste und die zweite Kopplung ausgebildet sind, am Funktionselektrodenpol eine zumindest teilweise destruktive Interferenz von elektromagnetischen Radiofrequenz-Wellen zu bewirken, die in der mindestens einen ersten Ader und der mindestens einen zweiten Ader des ersten Funktionsleiters geführt sind.

3. Medizinisches Gerät nach einem der vorstehenden Ansprüche, bei dem die mindestens eine erste Ader und die mindestens eine zweite Ader des ersten Funktionsleiters in mindestens einem dritten, vom ersten und zweiten verschiedenen und mit einem geringeren Abstand als diese von einem proximalen Ende der elektrischen Leitung angeordneten Längsabschnitt eine dritte Kopplung aufweisen, die geeignet ist, in der mindestens einen zweiten Ader geführte elektromagnetische Radiofrequenz-Welle zumindest teilweise in die mindestens eine erste Ader des ersten Funktionsleiters einzukoppeln.

4. Medizinisches Gerät nach einem der Ansprüche 1 bis 2, bei dem die mindestens eine erste Ader und mindestens eine Ader des zweiten Funktionsleiters in mindestens einem dritten, vom ersten und zweiten verschiedenen und mit einem geringeren Abstand als diese von einem proximalen Ende der elektrischen Leitung angeordneten Längsabschnitt eine dritte Kopplung aufweisen, die die geeignet ist, in der mindestens einen zweiten Ader geführte elektromagnetische Radiofrequenz-Welle zumindest teilweise in die mindestens eine erste Ader des ersten Funktionsleiters einzukoppeln.

5. Medizinische Gerät nach einem der vorstehenden Ansprüche, bei dem die mindestens eine erste Ader des ersten Funktionsleiters oder die mindestens eine zweite Ader in der elektrischen Leitung mindestens einem Phasenschieber-Bauelement zugeführt ist, das dem Elektrodenpol vorgeschaltet ist.

6. Medizinische Gerät nach einem der vorstehenden Ansprüche, bei dem die Kopplung der mindestens einen ersten und der mindestens einen zweiten Ader durch einen elektrischen Schleifkontakt erfolgt.

7. Medizinische Gerät nach einem der vorstehenden Ansprüche, bei dem die Kopplung der mindestens einen ersten und der mindestens einen zweiten Ader kapazitiv ist.

8. Medizinische Gerät nach einem der vorstehenden Ansprüche, bei dem die mindestens eine erste Ader des ersten Funktionsleiters mit mindestens einer Ader des zweiten Funktiosnleiters verdrillt ist.

9. Medizinische Gerät nach einem der vorstehenden Ansprüche, bei dem die mindestens eine erste Ader des ersten Funktionsleiters wendelförmig um mindestens eine Ader des zweiten Funktiosnleiters gewickelt ist und sie einhüllt.

10. Medizinische Gerät nach einem der vorstehenden Ansprüche, bei dem die mindestens eine erste Ader des ersten Funktionsleiters zumindest abschnittsweise als Seilleitung ausgebildet ist.

11. Medizinische Gerät nach einem der vorstehenden Ansprüche, bei dem die mindestens eine erste Ader des ersten Funktionsleiters und mindestens eine Ader des zweiten Funktionsleiters aus unterschiedlichen Materialien gefertigt sind.

## Claims

1. A temporarily or permanently implantable medical device comprising at least one elongate electric lead having at least two function conductors, which are each multi-stranded, each function conductor being connected to at least one respective function electrode pole to deliver treatment signals or to record diagnostic signals, in which device
- a first of the function conductors (100) has at least one first strand, which, in a course of the longitudinal extension of the first function conductor in at least one first longitudinal portion, which forms only part of the longitudinal extension of the function conductor, has a first coupling to a second of the function conductors (200), which coupling is configured to couple, at least in part, electromagnetic radio-frequency waves guided in the second function conductor into the at least one first strand of the first function conductor and, on account of an inherent physical separation of at least one second strand of the first function conductor, which second strand is different from the first strand, from the second function conductor, not into the at least one second strand, which is different from the first strand, of the first function conductor, and in which
- the at least one first strand in the course of the longitudinal extension of the first function conductor in at least one second longitudinal portion different from the first longitudinal portion has a second coupling to the at least one second strand of the first function conductor, which coupling is suitable for coupling, at least in part, electromagnetic radio-frequency waves guided in the at least one first strand into the at least one second strand of the first function conductor, and wherein
- the at least one first strand of the first function conductor is guided in the first longitudinal portion at a shorter distance from the second function conductor, suitable for producing the first coupling, than outside the first longitudinal portion of the first function conductor.

2. The medical device according to Claim 1, in which the first and the second coupling are configured to produce an at least partially destructive interference of electromagnetic radio-frequency waves at the function electrode pole, said electromagnetic radio-frequency waves being guided in the at least one first strand and the at least one second strand of the first function conductor.

3. The medical device according to any one of the preceding claims, in which the at least one first strand and the at least one second strand of the first function conductor in at least one third longitudinal portion, different from the first and second and arranged at a shorter distance than these from a proximal end of the electric lead, have a third coupling that is suitable for coupling, at least in part, electromagnetic radio-frequency waves guided in the at least one second strand into the at least one first strand of the first function conductor.

4. The medical device according to any one of Claims 1 or 2, in which the at least one first strand and at least one strand of the second function conductor in at least one third longitudinal portion, different from the first and the second and arranged at a shorter distance than these from a proximal end of the electric lead, have a third coupling that is suitable for coupling, at least in part, electromagnetic radio-frequency waves guided in the at least one second strand into the at least one first strand of the first function conductor.

5. The medical device according to any one of the preceding claims, in which the at least one first strand of the first function conductor or the at least one second strand is fed in the electric lead to at least one phase shifter component, which is arranged upstream of the electrode pole.

6. The medical device according to any one of the preceding claims, in which the coupling of the at least one first and the at least one second strand is provided by an electric sliding contact.

7. The medical device according to any one of the preceding claims, in which the coupling of the at least one first and the at least one second strand is capacitive.

8. The medical device according to any one of the preceding claims, in which the at least one first strand of the first function conductor is twisted with at least one strand of the second function conductor.

9. The medical device according to any one of the preceding claims, in which the at least one first strand of the first function conductor is wound in a helical manner around at least one strand of the second function conductor and enwraps said second function conductor.

10. The medical device according to any one of the preceding claims, in which the at least one first strand of the first function conductor is configured at least in some portions as a cable lead.

11. The medical device according to any one of the preceding claims, in which the at least one first strand of the first function conductor and at least one strand of the second function conductor are made of different materials.

## Revendications

1. Appareil médical implantable de manière temporaire ou permanente avec au moins une ligne électrique rallongée, avec au moins deux conducteurs fonctionnels qui sont chacun multifilaire, et parmi eux, chaque conducteur fonctionnel est relié avec au moins un pôle d'électrode fonctionnel correspondant, pour la délivrance de signaux de thérapie ou pour la saisie de signaux de diagnostic, dans lequel
- un premier parmi les conducteurs fonctionnels (100) présente au moins un premier fil qui, sur le trajet de l'extension longitudinale du premier conducteur fonctionnel dans au moins une première portion longitudinale, qui ne forme qu'une partie de l'extension longitudinale du conducteur fonctionnel, présente un premier couplage avec un deuxième conducteur fonctionnel (200) qui est configuré pour ne pas accoupler des ondes de radiofréquences électromagnétiques véhiculées dans le deuxième conducteur fonctionnel au moins partiellement dans l'au moins un premier fil du premier conducteur fonctionnel, et en raison d'une séparation spatiale inhérente, au moins un des premiers des divers deuxièmes fils du premier conducteur fonctionnel du deuxième conducteur fonctionnel, dans l'au moins un des premiers divers deuxièmes fils du premier conducteur fonctionnel, et dans lequel
- l'au moins un premier fil présente un deuxième couplage avec au moins un deuxième fil du premier conducteur fonctionnel sur le trajet de l'extension longitudinale du premier conducteur fonctionnel dans au moins une deuxième portion longitudinale, différente de la première portion longitudinale, couplage qui est approprié pour accoupler des ondes de radiofréquences électromagnétiques véhiculées dans l'au moins un premier fil au moins partiellement dans l'au moins un deuxième fil du premier conducteur fonctionnel, et où
- l'au moins un premier fil du premier conducteur fonctionnel est mené dans la première portion longitudinale avec un espace minime, approprié pour l'établissement du premier couplage par rapport au deuxième conducteur fonctionnel, extérieurement par rapport à la première portion longitudinale du premier conducteur fonctionnel.

2. Appareil médical selon la revendication 1, dans lequel les premier et deuxième couplages sont conçus pour provoquer une interférence d'ondes de radiofréquences électromagnétiques au moins partiellement destructrices au niveau du pôle d'électrode fonctionnelle, ondes qui sont véhiculées dans l'au moins un premier fil et dans l'au moins un deuxième fil du premier conducteur fonctionnel.

3. Appareil médical selon l'une des revendications précédentes, dans lequel l'au moins un premier fil et l'au moins un deuxième fil du premier conducteur fonctionnel présentent un troisième couplage dans au moins une troisième portion longitudinale, différente des première et deuxième portions, et agencée avec un espace plus faible que celles-ci avec l'extrémité proximale de ligne électrique, couplage qui est approprié pour accoupler l'onde de radiofréquence électromagnétique véhiculée dans l'au moins un deuxième fil au moins partiellement dans l'au moins un premier fil du premier conducteur fonctionnel.

4. Appareil médical selon l'une des revendications 1 à 2, dans lequel l'au moins un premier fil et au moins un fil du deuxième conducteur fonctionnel présentent un troisième couplage dans au moins une troisième portion longitudinale, différente des première et deuxième portions et agencée avec un espace plus faible que celles-ci par rapport à une extrémité proximale de la ligne électrique, couplage qui est approprié pour accoupler l'onde de radiofréquence électromagnétique véhiculée dans l'au moins un second fil au moins partiellement dans l'au moins un premier fil du premier conducteur fonctionnel.

5. Appareil médical selon l'une des revendications précédentes, dans lequel l'au moins un premier fil du premier conducteur fonctionnel, ou l'au moins un deuxième fil dans la ligne électrique sont menés vers un composant déphaseur qui est placé avant le pôle d'électrode.

6. Appareil médical selon l'une des revendications précédentes, dans lequel le couplage de l'au moins un premier fil et de l'au moins un deuxième fil s'effectue par un contact balai électrique.

7. Appareil médical selon l'une des revendications précédentes, dans lequel le couplage de l'au moins un premier fil et de l'au moins un deuxième fil est capacitif

8. Appareil médical selon l'une des revendications précédentes, dans lequel l'au moins un premier fil du premier conducteur fonctionnel est torsadé avec au moins un fil du deuxième conducteur fonctionnel.

9. Appareil médical selon l'une des revendications précédentes, dans lequel l'au moins un premier fil du premier conducteur fonctionnel est enroulé sous forme hélicoïdale autour d'au moins un fil du deuxième conducteur fonctionnel et l'enveloppe.

10. Appareil médical selon l'une des revendications précédentes, dans lequel l'au moins un premier fil du premier conducteur fonctionnel est conçu au moins sur des portions sous la forme d'une ligne torsadée.

11. Appareil médical selon l'une des revendications précédentes, dans lequel l'au moins un premier fil du premier conducteur fonctionnel et au moins un fil du deuxième conducteur fonctionnel sont fabriqués dans des matériaux différents.
